# EUROPEAN PATENT APPLICATION

(11) **EP 1 946 743 A2**
(43) Date of publication of application: **23.07.2008**
(21) Application number: 08100607.4
(22) Date of filing: 17.01.2008
(51) Int. Cl.: A61K 8/34, A61Q 5/00, A61Q 19/00

(54) **Humectant Composition**

(30) Priority: 17.01.2007 JP 2007008321
(71) Applicant: Takasago International Corporation, Tokyo (JP)
(72) Inventor: Yamamoto, Tetsuya Corp. Research&Dev. Division, Hiratsuka-shi, Kanagawa (JP); Ishida, Kenya Corp. Research&Dev. Division, Hiratsuka-shi, Kanagawa (JP)
(74) Representative: Jones, Helen M.M.

(57) **Abstract**

The present invention relates to a humectant composition containing, as component (A), at least one member selected from a menthol derivative represented by the following formula (1) and p-menthane-3,8-diol: 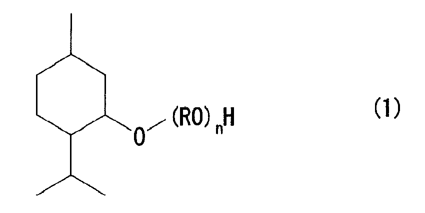 in which R is an alkylene group having 2 to 5 carbon atoms which may be substituted with at least one member selected from the group consisting of a methyl group and a hydroxyl group, and n is an integer of 1 to 3. The humectant composition of the present invention provides an excellent moisture-retention effect.

## Description

### FIELD OF THE INVENTION

The present invention relates to a humectant composition and a dermatological external agent containing the humectant composition incorporated therein.

### BACKGROUND OF THE INVENTION

In order to retain the efficacy of an external agent for application to skin, which is represented by a fragrance or cosmetic product, a toiletry product, a bath agent, a pharmaceutical or the like, and to keep a feeling of use thereof, it is necessary to maintain a humidity of the external agent. As the humectants for that purpose, there have been conventionally used ethylene glycol, propylene glycol, glycerol, 1,3-butanediol, sorbitol and the like. Furthermore, with the diversification of product needs, new humectants such as sodium pyrrolidonecarboxylate and sodium hyaluronate have been developed (non-patent document 1).

Non-Patent Document 1: Shin Keshohingaku (New Cosmetic Science), 2nd edition, pages 152 to 156, edited by Takeo Mitsui, published by Nanzando on January 18, 2001

### SUMMARY OF THE INVENTION

An object of the invention is to provide a new humectant composition for a dermatological external agent.

As a result of intensive studies on moisture-retention effect, it has been found that moisture retention can be maintained for an extremely long period of time when at least one member selected from a specific menthol derivative and p-menthane-3,8-diol is contained, thereby achieving the invention. Namely, the invention relates to a humectant composition containing at least one member selected from a specific menthol derivative and p-menthane-3,8-diol, and a dermatological external agent containing the humectant composition incorporated therein.

According to the invention, a humectant composition having an excellent moisture-retention effect can be provided, and a dermatological external agent containing the composition can maintain a humidity for a long period of time.

### DETAILED DESCRIPTION OF THE INVENTION

Namely, the present invention relates to the following (1) to (10).
(1) A humectant composition comprising, as component (A), at least one member selected from a menthol derivative represented by the following formula (1) and p-menthane-3,8-diol: 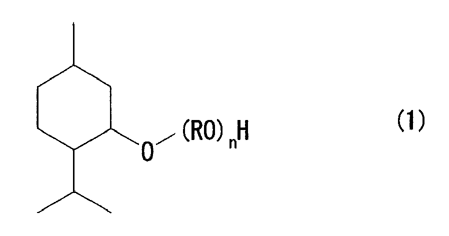 wherein R is an alkylene group having 2 to 5 carbon atoms which may be substituted with at least one member selected from the group consisting of a methyl group and a hydroxyl group, and n is an integer of 1 to 3.
(2) The humectant composition according to (1), wherein the menthol derivative is a 1-menthol derivative represented by the following formula (2): 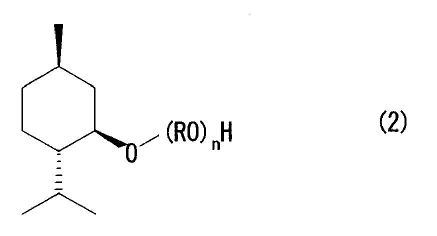 wherein R is an alkylene group having 2 to 5 carbon atoms which may be substituted with at least one member selected from the group consisting of a methyl group and a hydroxyl group, and n is an integer of 1 to 3.
(3) The humectant composition according to (1) or (2), wherein the component (A) is at least one member selected from the group consisting of 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, 2-(2-1-menthoxyethyl)ethanol and p-menthane-3,8-diol.
(4) The humectant composition according to any one of (1) to (3), which further comprises, as component (B), at least one member selected from ceramides represented by the following formula (3): 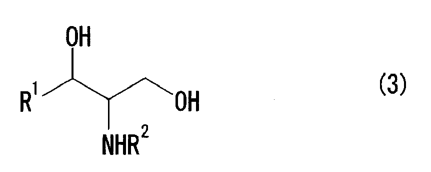 wherein R¹ is an alkyl group having 9 to 23 carbon atoms which may have a hydroxyl group and/or a double bond, and R² is an acetyl group or an acyl group having 14 to 30 carbon atoms which may have a hydroxyl group and/or a double bond.
(5) The humectant composition according to any one of (1) to (4), which further comprises, as additional component (C), at least one member selected from the group consisting of ethylene glycol, propylene glycol, glycerol, diethylene glycol, dipropylene glycol, 1,3-butylene glycol, polyethylene glycol, sorbitol, maltitol, sodium lactate, sodium 2-pyrrolidone-5-carboxylate, chondroitin sulfate, hyaluronic acid, sodium hyaluronate, collagen, coix seed extract and a chitosan derivative.
(6) A dermatological external agent comprising 0.0001% to 10% by weight of the humectant composition according to any one of (1) to (5) incorporated therein.
(7) The dermatological external agent according to (6), which is a fragrance or cosmetic product, a toiletry product, a bath agent or a pharmaceutical.
(8) A fragrance composition comprising 0.0001% to 20% by weight of the humectant composition according to any one of (1) to (5) incorporated therein.
(9) A dermatological external agent comprising 0.0001% to 10% by weight of the fragrance composition according to (8) incorporated therein.
(10) The dermatological external agent according to (9), which is a fragrance or cosmetic product, a toiletry product, a bath agent or a pharmaceutical.

As the component (A) used as an active ingredient in the present invention, namely as the menthol derivative represented by the formula (1) and p-menthane-3,8-diol, 3-(menthoxy)propane-1,2-diol, 2-(menthoxy)ethane-1-ol, 3-(menthoxy)propane-1-ol, 2-methyl-3-(menthoxy)propane-1,2-diol, 2-(2-menthoxyethyl)ethanol, p-menthane-3,8-diol and the like may be specifically mentioned.

Furthermore, in the humectant composition of the invention, the menthol derivative of the formula (1) is preferably a 1-menthol derivative represented by the formula (2): 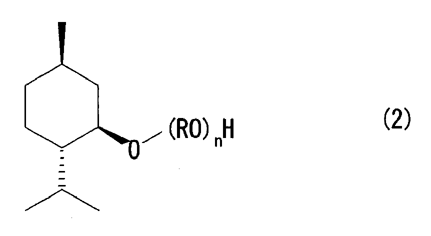 wherein R is an alkylene group having 2 to 5 carbon atoms which may be substituted with at least one member selected from the group consisting of a methyl group and a hydroxyl group, and n is an integer of 1 to 3.

Specific examples of the 1-menthol derivative represented by the formula (2) include 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol and 2-(2-1-menthoxyethyl)ethanol.

These compounds may be used either solely or as a combination of two or more thereof. These compounds are colorless and almost odorless oily substances, and extremely high in compatibility with ordinary dermatological external agents.

Of these compounds, 3-(1-menthoxy)propane-1,2-diol and p-menthane-3,8-diol are particularly preferable, since they are excellent in moisture retention and excellent in terms of moisture-retention effect at the time when used in a dermatological external agent such as a fragrance or cosmetic product, a toiletry product, a bath agent or a pharmaceutical.

It has already been described in JP-A-58-88334, JP-A-47-16647 and the like that 3-(1-menthoxy)propane-1,2-diol represented by the formula (1) has a cool feeling effect, and further, it has already been described in JP-A-2002-88391 and the like that it has a fragrance-retention effect. However, there has been no report suggesting that the menthol derivative of the above-mentioned formula (1) and/or p-menthane-3,8-diol have a moisture-retention effect and have the effect of significantly enhancing moisture-retention effect of various dermatological external agents.

The compounds represented by the above-mentioned formulae (1) and/or (2), which are used in the invention, are known compounds. For example, 3-(menthoxy)propane-1,2-diol can be synthesized in accordance with the method described in JP-A-58-88334; 2-(menthoxy)ethane-1-ol can be synthesized in accordance with the method described in British Patent 1,315,626; 3-(menthoxy)propane-1-ol can be synthesized in accordance with the method described in JP-A-2001-294546; 2-methyl-3-(menthoxy)propane-1,2-diol can be synthesized in accordance with the method described in JP-A-9-217083; 2-(2-menthoxyethyl)ethanol can be synthesized in accordance with the method described in JP-A-2005-343915; and p-menthane-3,8-diol can be synthesized in accordance with the method described in JP-A-11-130710, respectively.

In the humectant composition of the present invention, at least one member selected from ceramides represented by the formula (8): 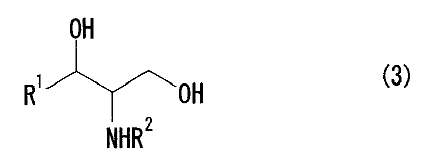 wherein R¹ is an alkyl group having 9 to 23 carbon atoms which may have a hydroxyl group and/or a double bond, and R² is an acetyl group or an acyl group having 14 to 30 carbon atoms which may have a hydroxyl group and/or a double bond, may be further added as component (B) to thereby prepare a humectant composition having an enhanced moisture-retention effect.

Although the ceramides represented by the above-mentioned formula (3) are not particularly limited, specific examples thereof include 2-acetylaminooctadecane-1,3-diol, 2-tetradecanoylaminooctadecane-1,3-diol, 2-hexadecanoylaminooctadecane-1,3-diol, 2-octadecanoylaminooctadecane-1,3-diol, 2-eicosanoylaminooctadecane-1,3-diol, 2-oleoylaminooctadecane-1,3-diol, 2-linolenoylaminooctadecane-1,3-diol, 2-(2-hydroxyhexadecanoyl)aminooctadecane-1,3-diol, 2-(3-hydroxyhexadecanoyl)aminooctadecane-1,3-diol, 2-acetylaminohexadecane-1,3-diol, 2-tetradecanoylaminohexadecane-1,3-diol, 2-hexadecanoylaminohexadecane-1,3-diol, 2-octadecanoylaminohexadecane-1,3-diol, 2-eicosanoylaminohexadecane-1,3-diol, 2-oleoylaminohexadecane-1,3-diol, 2-linolenoylaminohexadecane-1,3-diol, 2-(2-hydroxyhexadecanoyl)aminohexadecane-1,3-diol, 2-acetylaminooctadecane-1,3,4-triol, and 2-octadecanoylaminooctadecane-1,3,4-triol.

In particular, as the ceramide represented by the formula (3), preferably used is an optically-active natural ceramide having a D-erythro structure, which is represented by the following formula (4): 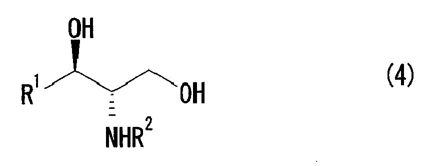 wherein R¹ is an alkyl group having 9 to 23 carbon atoms which may have a hydroxyl group and/or a double bond, and R² is an acetyl group or an acyl group having 14 to 30 carbon atoms which may have a hydroxyl group and/or a double bond.

Although the compound represented by the formula (4) are not particularly limited, specific examples thereof include (2S,3R)-2-acetylaminooctadecane-1,3-diol, (2S,3R)-2-tetradecanoylaminooctadecane-1,3-diol, (2S,3R)-2-hexadecanoylaminooctadecane-1,3-diol, (2S,3R)-2-octadecanoylaminooctadecane-1,3-diol, (2S,3R)-nonadecanoylaminooctadecane-1,3-diol, (2S,3R)-2-eicosanoylaminooctadecane-1,3-diol, (2S,3R)-2-oleoylaminooctadecane-1,3-diol, (2S,3R)-2-linolenoylaminooctadecane-1,3-diol, (2S,3R)-2-(2-hydroxyhexadecanoyl)aminooctadecane-1,3-diol, (2S,3R)-2-(3-hydroxyhexadecanoyl)aminooctadecane-1,3-diol, (2S,3R)-2-acetylaminohexadecane-1,3-diol, (2S,3R)-2-tetradecanoylaminohexadecane-1,3-diol, (2S,3R)-2-hexadecanoylaminohexadecane-1,3-diol, (2S,3R)-2-octadecanoylaminohexadecane-1,3-diol, (2S,3R)-2-nonadecanoylaminohexadecane-1,3-diol, (2S,3R)-2-eicosanoylaminohexadecane-1,3-diol, (2S,3R)-2-oleoylaminohexadecane-1,3-diol, (2S,3R)-2-linolenoylaminohexadecane-1,3-diol, (2S,3R)-2-(2-hydroxyhexadecanoyl)aminohexadecane-1,3-diol, (2S,3S,4R)-2-acetylaminooctadecane-1,3,4-triol, and (2S,3S,4R)-2-octadecanoylaminooctadecane-1,3,4-triol.

Of these, (2S,3R)-2-octadecanoylaminooctadecane-1,3-diol and (2S,3R)-2-acetylaminooctadecane-1,3-diol are particularly preferred.

In the humectant composition of the invention, the ceramides represented by the formula (3) or the formula (4) can be either used solely or as a combination of two or more thereof.

The ceramides represented by the formula (3) or the formula (4) are hardly-soluble materials, and they are scarcely soluble in water. Furthermore, they and hard to dissolve into oily components at room temperature under atmospheric pressure, thereby precipitating fine crystals or amorphous solid matter.

Consequently, it is preferable to solubilize the ceramides represented by the formula (3) or the formula (4) for use thereof, and they can be solubilized in accordance with the methods described in JP-A-11-12118, JP-A-2001-348320, JP-A-2004-331595 and the like.

In the humectant composition of the present invention, at least one member selected from the group consisting of ethylene glycol, propylene glycol, glycerol, diethylene glycol, dipropylene glycol, 1,3-butylene glycol, polyethylene glycol, sorbitol, maltitol, sodium lactate, sodium 2-pyrrolidone-5-carboxylate, chondroitin sulfate, hyaluronic acid, sodium hyaluronate, collagen, coix seed extract and a chitosan derivative may be further added as additional component (C) to thereby prepare a humectant composition having a further enhanced moisture-retention effect.

In the humectant composition of the invention, the amounts of the menthol derivative and/or p-menthane-3,8-diol as component (A), the ceramide as component (B) and additional component (C) incorporated in the fragrance composition or the dermatological external agent and methods for applying them can be appropriately and optimally selected depending on the kind of fragrance composition or dermatological external agent in which they are incorporated, intended purpose thereof and the like. When component (A) is incorporated in a fragrance composition, the amount thereof is preferably from 0.0001% to 20% by weight, and more preferably from 0.001% to 10% by weight, based on the total amount of the fragrance composition. When the fragrance composition containing component (A) is incorporated in a dermatological external agent, usually, it is preferably employed at a concentration of 0.0001% to 10% by weight, particularly 0.001% to 3% by weight, based on the total composition of the dermatological external agent. Further, when component (B) is incorporated in the fragrance composition, the amount thereof is preferably from 0.01% to 5% by weight, and more preferably from 0.1% to 1% by weight, based on the total amount of the fragrance composition. When the fragrance composition containing component (B) is incorporated in a dermatological external agent, usually, it is preferably employed at a concentration of 0.01% to 5% by weight, particularly 0.1% to 1% by weight, based on the total composition of the dermatological external agent. Furthermore, when component (C) is incorporated in the fragrance composition, the amount thereof is preferably from 0.01% to 5% by weight, and more preferably from 0.1% to 1% by weight, based on the total amount of the fragrance composition. When the fragrance composition containing component (C) is incorporated in a dermatological external agent, usually, it is preferably employed at a concentration of 0.001% to 5% by weight, particularly 0.1% to 1% by weight, based on the total composition of the dermatological external agent.

On the other hand, when directly incorporated in the dermatological external agent, they can be incorporated in any amounts depending on the kind of the dermatological external agent and intended purpose thereof. However, when component (A) is incorporated in the dermatological external agent, it is preferably employed at a concentration of 0.0001% to 10% by weight, particularly 0.0005% to 1% by weight, based on the total composition of the dermatological external agent. Further, when component (B) is incorporated in the dermatological external agent, it is preferably employed at a concentration of 0.0001% to 5% by weight, particularly 0.0005% to 1% by weight, based on the total composition of the dermatological external agent. Furthermore, when component (C) is incorporated in the dermatological external agent, it is preferably employed at a concentration of 0.0001% to 50% by weight, particularly 0.0005% to 30% by weight, based on the total composition of the dermatological external agent.

There is no particular limitation on the fragrances which can be used in the fragrance composition incorporated in the humectant composition of the invention, and both of synthetic aroma chemicals and natural aroma chemicals can be used. For example, a wide variety of fragrance components as described in Arctander S., Perfume and Flavor Chemicals, published by the author, Montclair, N. J. (U. S. A.) in 1969, can be used.

The above-mentioned fragrances and recent typical fragrances which can be used in the fragrance composition of the invention are described below. Examples of the typical natural aroma chemicals include natural essential oils such as anise seed, ylang ylang, elemi, orris, orange, galbanum, clary sage, clove, coriander, sandalwood, citronella, cinnamon, spearmint, cedarwood, geranium, celery, tangerine, tonka been, nerori, violet, patchouli, peach, vetiver, petitgrain, peppermint, Peru balsam, bergamot, eucalyptus, lilac, raspberry, lavender, lily of the valley, lemon, lemongrass, lime and rose; and animal aroma chemicals such as ambergris, castoreum, civet and musk.

Examples of typical synthetic aroma chemicals include hydrocarbons such as pinene, limonene, caryophyllene, longifolene and myrcene; alcohols such as cis-3-hexenol, Levosandol (Takasago International Corporation), p-t-butylocyclohexanol, citronellol, geraniol, nerol, linalool, dihydrolinalool, tetrahydrolinalool, dihydromyrcenol, tetrahydromyrcenol, menthol, terpineol, borneol, isoborneol, isocanphyl cyclohexanol, farnesol, cedorol, benzyl alcohol, α-phenylethyl alcohol, β-phenylethyl alcohol, phenoxyethyl alcohol, cinnamic alcohol, amylcinnamic alcohol, thymol and eugenol; ethers such as cineol, estragole, β-naphthol methyl ether, β-naphthol ethyl ether, diphenyl oxide, cedorol methyl ether, isoamyl phenylethyl ether, Ambroxan (Kao Corporation), Grisalva (IFF), rose oxide, dihydroose oxide, limonene oxide, menthofuran and Ambercore (Kao Corporation); aldehydes such as C9 to C12 aldehydes, citronellal, citral, hydroxycitronellal, dimethyltetrahydrobenzaldehyde, Myrac aldehyde (IFF), Kovanol (Takasago International Corporation), Vernaldehyde (Givaudan SA), benzaldehyde, cyclamen aldehyde, Suzaral (Takasago International Corporation), Lilial (Givaudan SA), cinnamic aldehyde, methylcinnamic aldehyde, amylcinnamic aldehyde, hexylcinnamic aldehyde, vanillin, ethylvanillin heliotropin and Heliobouquet (Takasago International Corporation); ketones such as cis-jasmone, dihydrojasmine, methyl dihydrojasmonate (Hedion; Firmenich SA), cyclotene, damascenone, damascone, dynascone, ionone, methylionone, irone, Cashmeran (IFF), Iso E Super (IFF), carvone, menthone, acetylcedrene, iso-longifolanone, raspberry ketone, acetophenone and benzophenone; esters such as γ-undecalactone, coumarin, linalyl formate, citronellyl formate, linalyl acetate, citronellyl acetate, geranyl acetate, terpenyl acetate, cedryl acetate, p-t-butylcyclohexyl acetate (Veltenex; IFF), 2-t-butylcyclohexyl acetate (Veldox; IFF), tricyclodecenyl acetate (Erica acetate; Takasago International Corporation), benzyl acetate, phenylallyl acetate, styralyl acetate, isoamyl acetate, rosephenone, dimethylbenzylcarbinyl acetate, Jasmal (IFF), benzyl benzoate, benzyl salicylate, hexyl salicylate, methyl atrarate, methyl anthranilate, dimethyl anthranilate, ethyl anthranilate, Auranthiol (Givaudan SA), ethyl trimethylcyclohexanecarboxylate (THESARON; Takasago International Corporation) and Fruitate (Kao Corporation); and musks such as muscone, muscol, civetone, cyclopentadecanone, cyclohexadecanone (Ambreton; Takasago International Corporation), cyclopentadecanolide, 10-oxahexadecanolide, ethylene brassylate (Musk T; Takasago International Corporation), ethylene dodecanedioate, Celestolid (IFF), Tonalid (PFW), Galaxolide (IFF), Traseolide (Quest International) and Phantolid (PFW). These fragrances may be used either solely or as a blended fragrance by blending two or more thereof.

Further, one or two or more of ordinarily-used fragrance retention agents may be blended, and it is also possible to use them in combination with, for example, propylene glycol, glycerol, hexylene glycol, dipropylene glycol, benzyl benzoate, triethyl citrate, diethyl phthalate, Hercolyn (methyl abietate) or the like.

The dermatological external agent of the invention is prepared by blending various bases and the humectant composition of the invention or the fragrance composition containing the humectant composition of the invention incorporated therein. Since the humectant composition of the invention and the fragrance composition containing the humectant composition of the invention incorporated therein are excellent in compatibility, it is possible to use bases having any form of solid, liquid, emulsion, gel, foam and the like, so long as they are a commonly used base for a dermatological external agent. Examples of the bases include water; alcohols such as ethanol, isopropyl alcohol, cetyl alcohol and stearyl alcohol; polyhydric alcohols such as propylene glycol, glycerol and 1,3-butanediol; esters such as isopropyl myristate, propylene glycol monostearate and glycerol tricaprate; animal and vegetable fats and oils such as castor oil, olive oil, lanolin, squalane and spermaceti wax; mineral oils such as paraffin and liquid paraffin; fatty acids such as lauric acid, 12-hydroxystearic acid and behenic acid; silicone oils, aerosol propellants; and solid carriers such as silica, talc and synthetic resin powder.

Further, in the dermatological external agent of the invention which contains the humectant composition of the invention or the fragrance composition containing the humectant composition of the invention incorporated therein, other components ordinarily used, such as various cosmetic components, a humectant, a thickener, an ultraviolet absorber, a preservative agent, an antioxidant, a coloring material and a surfactant may be arbitrarily contained. The dermatological external agent of the invention may be in any form, and a desired preparation form such as a solution system, a solubilized system, a emulsion system, a powder dispersion system, a water-oil two-phase system or water-oil-powder three-phase system can be taken by blending the humectant composition of the invention or the fragrance composition containing the humectant composition of the invention incorporated therein with the above-mentioned bases and other components appropriately selected. The dermatological external agent of the invention may also take any product form, and can be used as facial cosmetics such as an aerosol, a spraying agent, a lotion, an emulsion and a facial mask; makeup cosmetics such as a foundation, a lip stick and an eye shadow; body cosmetics; aromatic cosmetics; skin cleaners such as a makeup remover and a body shampoo; and ointments; and the like. As a method for preparing the preparation, there can be employed an ordinary method, and examples thereof include methods described in Koshohin Kagaku (Fragrance Science)-Riron to Jissai (Theory and Practice), written by Takeo Murata and Hiroshi Hirota, Fragrance Journal (Sept. 25, 1990); Keshohin Seizai Jitsuyo Binran (Practical Manual of Cosmetic Preparations), Kokichi Hikime, Nikko Chemical Co., Ltd. (1982); and the like.

The dermatological external agent thus obtained may be applied to human skin by an ordinary method depending on the use thereof.

Further, the dermatological external agent of the invention also has an excellent cool feeling effect, so that it is useful in use requiring such a cool feeling effect. In such use, it is desirable to use 3-(1-menthoxy)propane-1,2-diol and p-menthane-3,8-diol among the menthol derivative and/or p-menthane-3,8-diol according to the invention.

### EXAMPLES

The invention will be illustrated in greater detail with reference to the following examples and test examples, but the invention should not be construed as being limited by these examples.

### Test Example 1

Process: Creams described in Table 1 were prepared, and each sample was thinly placed on a slide glass to prepare a test sample. After standing at room temperature (25°C/60% RH) for 6 hours, the weight of each sample was measured to assay the amount of water evaporation. Each sample was tested three times, and the water retention was determined from the average value thereof. The test results are shown in Table 2.

**Table 1: Cream**

| Component | Example 1 | Comparative Example 1 |
|---|---|---|
| Stearic Acid | 1.00 | 1.00 |
| Cholesteryl Isostearate | 2.00 | 2.00 |
| Jojoba Oil | 4.00 | 4.00 |
| Squalane | 8.00 | 8.00 |
| Sorbitan Sesquioleate | 0.80 | 0.80 |
| Polyoxyethylene Sorbitan Mono-stearate (20 E.O.) | 1.20 | 1.20 |
| 1,3-Butylene Glycol | 5.00 | 5.00 |
| Paraoxybenzoate | 0.25 | 0.25 |
| L-Arginine | 0.40 | 0.40 |
| Carboxyvinyl Polymer | 0.20 | 0.20 |
| Fragrance | 0.05 | 0.05 |
| 3-(Menthoxy)propane-1,2-diol | 3.00 | - |
| Purified Water | to 100.00 | to 100.00 |

**Table 2: Test Results**

| | Average Water Retention (%) |
|---|---|
| Example 1 | 25.4 |
| Comparative Example 1 | 21.0 |

As apparent from Table 2, it was confirmed that the sample (Example 1) in which 3-(menthoxy)propane-1,2-diol was incorporated had an excellent water-retention function.

### Test Example 2

Process: Emollient milks described in Table 3 were prepared, and each of them was applied to the inside of the forearm in an amount of 65 mg/cm². After standing for 10 minutes for drying, the conductance values were measured with time for 30 minutes (n=3).

The test results are shown in Table 4 (unit: µS).

**Table 3: Emollient Milk**

| Component | Example 2 | Comparative Example 2 |
|---|---|---|
| Stearic Acid | 1.00 | 1.00 |
| Cholesteryl Isostearate | 2.00 | 2.00 |
| Jojoba Oil | 4.00 | 4.00 |
| Squalane | 8.00 | 8.00 |
| Sorbitan Sesquioleate | 0.80 | 0.80 |
| Polyoxyethylene Sorbitan Mono-stearate (20 E.O.) | 1.20 | 1.20 |
| 1,3-Butylene Glycol | 5.00 | 5.00 |
| Paraoxybenzoate | 0.25 | 0.25 |
| L-Arginine | 0.40 | 0.40 |
| Carboxyvinyl Polymer | 0.20 | 0.20 |
| Fragrance | 0.05 | 0.05 |
| 3-(Menthoxy)propane-1,2-diol | 3.00 | - |
| Purified Water | to 100.00 | to 100.00 |

**Table 4: Test Results**

| | At the Start of Experiment | Elapsed Time after Application of Lotion | | | | | | |
|---|---|---|---|---|---|---|---|---|
| | | 5 | 6 | 7 | 10 | 15 | 25 | 30 |
| Example 2 | 39 | 329 | 392 | 347 | 272 | 207 | 161 | 94 |
| Comparative Example 2 | 31 | 323 | 272 | 222 | 174 | 145 | 125 | 69 |

As apparent from Table 4, it was confirmed that the sample (Example 2) in which 3-(menthoxy)propane-1,2-diol was incorporated had persistently high conductance values and showed an excellent moisture-retention function.

### Test Example 3

Each of the emollient milks described in Table 3 was applied to the inside of the forearm in the same manner in Test Example 2, and dried for 30 minutes. A drop of distilled water was placed on that site to conduct water supply for 10 seconds, followed by wiping off. Then, the conductance values were measured with time for 15 minutes (n=3).

The results are shown in Table 5.

**Table 5: Test Results**

| | Elapsed Time after Application of Water | | |
|---|---|---|---|
| | 5 | 10 | 15 |
| Example 2 | 169 | 94 | 69 |
| Comparative Example 2 | 73 | 58 | 39 |

As apparent from Table 5, the site to which the sample (Example 2) in which 3-(menthoxy)propane-1,2-diol was incorporated was applied showed a high water-retention function.

### Test Example 4

Process: Emollient milks having the same components as described in Table 3 with the exception that 3-(menthoxy)propane-1,2-diol was substituted by p-menthane-3,8-diol were prepared (Example 3 and Comparative Example 3). Each was applied to the inside of the forearm in an amount of 65 mg/cm², and evaluated in the same manner as in Test Example 3.

The results are shown in Table 6.

**Table 6: Test Results**

| | Elapsed Time after Application of Water | | |
|---|---|---|---|
| | 5 | 10 | 15 |
| Example 3 | 150 | 87 | 66 |
| Comparative Example 3 | 79 | 65 | 43 |

As apparent from Table 6, it was confirmed that p-menthane-3,8-diol has an excellent water-retention function.

### Preparation Example 1

(2S,3R)-2-Octadecanoylaminooctadecane-1,3-diol (a compound represented by the formula (4) wherein R¹ is C₁₅H₃₁ and R² is C₁₇H₃₅), (2S, 3R) -2-acetylaminooctadecane-1, 3-diol (a compound represented by the formula (4) wherein R¹ is C₁₅H₃₁ and R² is CH₃), cholesterol and cholesteryl 12-hydroxystearate were mixed at a weight ratio of 2:1:1:2. This mixture was dissolved in chloroform and homogenized. Then, the solvent was completely removed, followed by standing to cool in air to obtain a pasty mixture. The resulting mixture is a lipid composition having a liquid crystal structure.

### Preparation Example 2

Process: The lipid composition obtained in Preparation Example 1 was added in an amount of 0.4% by weight to a floral green type fragrance composition for cream having a high-class image, which is shown in Table 7, to prepare a fragrance composition for cream. According to a formulation of Table 8, 20 g of a model cream was prepared, and 0.2 g of the fragrance composition for cream was incorporated therein to prepare a cream (Example 4). Further, according to the formulation of Table 8, 20 g of the model cream was prepared, and 0.2 g of the fragrance composition for cream in which no lipid composition was added was incorporated therein to prepare cream 1 as Comparative Example 4.

**Table 7: Fragrance composition for cream**

| Component | Parts by Weight |
|---|---|
| Hydroxycitronellal (Laurinal: Takasago International Corporation) | 2.0 |
| Linalool | 2.0 |
| Linalyl Acetate | 3.0 |
| Nerolidol | 3.0 |
| Perfume Base With Rose Note (Takasago International Corporation) | 15.0 |
| Isocamphyl Cyclohexanol (Santalex: Takasago International Corporation) | 3.0 |
| Tonalid (PFW) | 1.0 |
| Blended Base (Takasago International Corporation) | 5.5 |
| Benzyl Salicylate | 7.0 |
| Citronellol | 1.0 |
| Galaxolide (IFF) | 4.0 |
| Methyl Dihydrojasmonate (Hedion: Firmenich SA) | 5.0 |
| Helional (IFF) | 14.5 |
| Dihydromyrcenol | 3.0 |
| Dipropylene Glycol | 10.0 |
| Farnesol | 1.5 |
| cis-3-Hexenyl Salicylate | 4.5 |
| Lilial (Givaudan SA) | 5.5 |
| 2-(Menthoxy)ethane-1-ol | 10.0 |

**Table 8: Cream**

| Component | Parts by Weight |
|---|---|
| Stearic Acid | 1.00 |
| Cholesteryl Isostearate | 2.00 |
| Jojoba Oil | 4.00 |
| Squalane | 8.00 |
| Sorbitan Sesquioleate | 0.80 |
| Polyoxyethylene Sorbitan Monostearate (20 E.O.) | 1.20 |
| 1,3-Butylene Glycol | 5.00 |
| Paraoxybenzoate | 0.25 |
| L-Arginine | 0.40 |
| Carboxyvinyl Polymer | 0.20 |
| Fragrance Composition for Cream | 1.00 |
| Purified Water | to 100.00 |

### Test Example 5

Process: The cream described in Table 8 was thinly placed on a slide glass to prepare a test sample. After standing at room temperature (25°C/60% RH) for 6 hours, the weight of each sample was measured to assay the amount of water evaporation. Each sample was tested three times, anc the water retention was determined from the average value thereof. The test results are shown in Table 9.

**Table 9: Test Results**

| | Average Water Retention (%) |
|---|---|
| Example 4 | 27.0 |
| Comparative Example 4 | 23.1 |

As apparent from Table 9, it was confirmed that the sample (Example 4) in which the fragrance composition containing 3-(menthoxy)propane-1,2-diol was incorporated had an excellent water-retention function.

### Example 5

According to a conventional method, 100 g of a conditioning shampoo was produced.

**Table 10: Conditioning Shampoo**

| Component | Parts by Weight |
|---|---|
| Sodium Polyoxyethylene Lauryl Ether Sulfate | 14.00 |
| Lauric Acid Amide Propylbetaine | 4.00 |
| Coconut Oil Fatty Acid Diethanolamide | 3.00 |
| Cationized Cellulose | 0.50 |
| Ethylene Glycol Distearate | 1.00 |
| Paraoxybenzoate | 0.25 |
| Citric Acid | qs |
| Sodium 2-Pyrrolidone-5-carboxylate | 0.50 |
| Fragrance | 0.50 |
| p-Menthane-3,8-diol | 1.00 |
| Purified Water | to 100.00 |

### Example 6

According to a conventional method, 100 g of a hair rinse was produced.

**Table 11: Hair Rinse**

| Component | Parts by Weight |
|---|---|
| Stearyltrimethylammonium Chloride | 1.00 |
| Cetanol | 3.00 |
| Methylpolysiloxane | 1.00 |
| Polyoxyethylene Stearyl Ether | 1.00 |
| Propylene Glycol | 5.00 |
| Paraoxybenzoate | 0.25 |
| Chondroitin Sulfate | 0.05 |
| Sodium Hydroxide | qs |
| Citric Acid | qs |
| Fragrance | 0.50 |
| 3-(Menthoxy)propane-1,2-diol | 0.01 |
| Purified Water | to 100.00 |

### Example 7

According to a conventional method, 100 g of a hair conditioner was produced.

**Table 12: Hair Conditioner**

| Component | Parts by Weight |
|---|---|
| Stearyltrimethylammonium Chloride | 0.50 |
| Distearyldimethylammonium Chloride | 1.50 |
| Jojoba Oil | 2.50 |
| Cetanol | 4.50 |
| Liquid Lanolin | 2.00 |
| Polyoxyethylene Stearyl Ether | 1.50 |
| Concentrated Glycerol | 7.00 |
| Paraoxybenzoate | 0.25 |
| Sodium Hydroxide | qs |
| Citric Acid | qs |
| Fragrance | 0.50 |
| 2-(Menthoxy)ethane-1,2-diol | 0.50 |
| (2S,3R)-Octadecanoylaminooctadecane-1,3-diol | 0.10 |
| Purified Water | to 100.00 |

### Example 8

According to a conventional method, 100 g of a hair tonic was produced.

**Table 13: Hair Tonic**

| Component | Parts by Weight |
|---|---|
| Sialid Extract | 2.00 |
| L-Menthol | 0.10 |
| Hinokitiol | 0.01 |
| Fragrance | 0.10 |
| Paraoxybenzoate | 0.20 |
| Polyoxyethylene Hydrogenated Caster Oil | 0.50 |
| 3-(Menthoxy)propane-1,2-diol | 0.02 |
| Purified Water | to 100.00 |

### Example 9

According to a conventional method, 100 g of a liquid bath agent was produced.

**Table 14: Liquid Bath Agent**

| Component | Parts by Weight |
|---|---|
| Dipropylene Glycol | 50.00 |
| 1,3-Butylene Glycol | 10.00 |
| Paraoxybenzoate | 0.20 |
| Fragrance | 1.00 |
| 3-(Menthoxy)propane-1,2-diol | 0.05 |
| Purified Water | to 100.00 |

### Example 10

According to a conventional method, 100 g of a liquid body soap was produced.

**Table 15: Liquid Body Soap**

| Component | Parts by Weight |
|---|---|
| Lauric Acid | 3.00 |
| Myristic Acid | 1.00 |
| Polyoxyethylene Lauryl Ether Acetic Acid | 2.00 |
| N-Cocoyl-L-glutamic Acid | 12.00 |
| Polyethylene Glycol 1000 | 3.00 |
| Concentrated Glycerol | 12.00 |
| Xanthan Gum | 1.00 |
| Silicic Anhydride | 0.02 |
| 10% Potassium Hydroxide Aqueous Solution | 0.80 |
| 10% Polyvinylpyrrolidone Aqueous Solution | 0.02 |
| Acrylamide-Acrylic Acid-Dimethyl Chloride-Dially Ammonium Copolymer | 3.00 |
| Sodium Aspartate | 0.05 |
| Carrageenan | 0.02 |
| Caster Oil | 0.30 |
| Crosslinking Type Polyether-Modified Silicone Mixture | 1.00 |
| Phenoxyethanol | 0.50 |
| Fragrance | 0.50 |
| 3-(Menthoxy)propane-1,2-diol | 0.50 |
| Purified Water | to 100.00 |

While the invention has been described in detail and with reference to specific embodiments thereof, it will be apparent to one skilled in the art that various changes and modifications can be made therein without departing from the scope thereof.

This application is based on Japanese patent application No. 2007-008321 filed January 17, 2007, the entire contents thereof being hereby incorporated by reference.

Further, all references cited herein are incorporated in their entireties.

## Claims

1. A humectant composition comprising, as component (A), at least one member selected from a menthol derivative represented by the following formula (1) and p-menthane-3,8-diol: 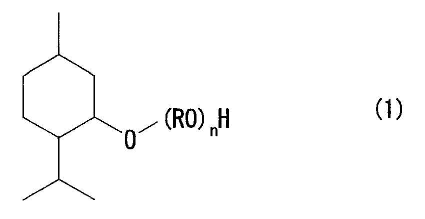 wherein R is an alkylene group having 2 to 5 carbon atoms which may be substituted with at least one member selected from the group consisting of a methyl group and a hydroxyl group, and n is an integer of 1 to 3.

2. The humectant composition according to claim 1, wherein the menthol derivative is a 1-menthol derivative represented by the following formula (2): 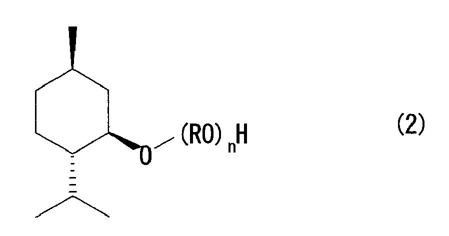 wherein R is an alkylene group having 2 to 5 carbon atoms which may be substituted with at least one member selected from the group consisting of a methyl group and a hydroxyl group, and n is an integer of 1 to 3.

3. The humectant composition according to claim 1, wherein the component (A) is at least one member selected from the group consisting of 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, 2-(2-1-menthoxyethyl)ethanol and p-menthane-3,8-diol.

4. The humectant composition according to claim 1, which further comprises, as component (B), at least one member selected from ceramides represented by the following formula (3): 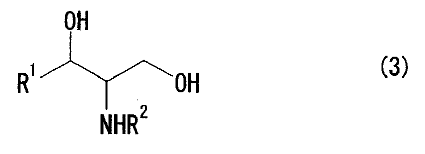 wherein R¹ is an alkyl group having 9 to 23 carbon atoms which may have a hydroxyl group and/or a double bond, and R² is an acetyl group or an acyl group having 14 to 30 carbon atoms which may have a hydroxyl group and/or a double bond.

5. The humectant composition according to claim 1, which further comprises, as additional component (C), at least one member selected from the group consisting of ethylene glycol, propylene glycol, glycerol, diethylene glycol, dipropylene glycol, 1,3-butylene glycol, polyethylene glycol, sorbitol, maltitol, sodium lactate, sodium 2-pyrrolidone-5-carboxylate, chondroitin sulfate, hyaluronic acid, sodium hyaluronate, collagen, coix seed extract and a chitosan derivative.

6. A dermatological external agent comprising 0.0001% to 10% by weight of the humectant composition according to claim 1 incorporated therein.

7. The dermatological external agent according to claim 6, which is a fragrance or cosmetic product, a toiletry product, a bath agent or a pharmaceutical.

8. A fragrance composition comprising 0.0001% to 20% by weight of the humectant composition according to claim 1 incorporated therein.

9. A dermatological external agent comprising 0.0001% to 10% by weight of the fragrance composition according to claim 8 incorporated therein.

10. The dermatological external agent according to claim 9, which is a fragrance or cosmetic product, a toiletry product, a bath agent or a pharmaceutical.
